# EUROPEAN PATENT APPLICATION

(11) **EP 1 149 839 A1**
(43) Date of publication of application: **31.10.2001**
(21) Application number: 00201545.1
(22) Date of filing: 28.04.2000
(51) Int. Cl.: C07J 7/00

(54) **Progesterone substantially free of delta14-pregnene-3,20-dione**

(71) Applicant: AZIENDE CHIMICHE RIUNITE ANGELINI FRANCESCO A.C.R.A.F. S.p.A., 00181 Roma (IT)
(72) Inventor: Giannangeli, Marilena, 00181 Roma (IT)
(74) Representative: Marchi, Massimo

(57) **Abstract**

Progesterone substantially free of Δ¹⁴-pregnene-3,20-dione and process for obtaining the same.

## Description

The present invention relates to progesterone substantially free of Δ¹⁴-pregnene-3,20-dione.

It is known that progesterone, or Δ⁴-pregnene-3,20-dione, of formula (I) 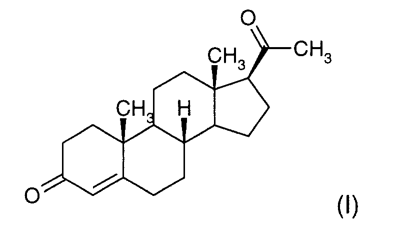 is prepared by oxidation according to the Oppenhauer technique ("Rec. Trav. Chim.", 56, 137, 1937) of pregnenolone, or Δ⁵-pregnen-3β-ol-20-one, of formula (II)

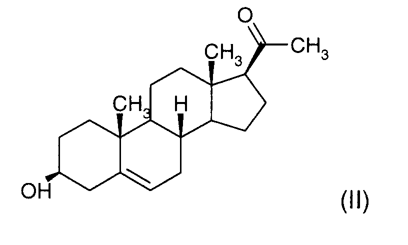

However, since the abovementioned starting compound (II) contains Δ¹⁴-pregnene-3,20-dione, of formula (III) 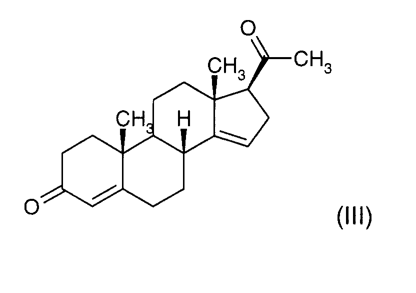 the progesterone (I) obtained is impure.

Thus, still there is a need for a source of progesterone (I) which is free of the abovementioned compound of formula (III).

Now, it has been surprisingly found that this can be achieved by recrystallizing the starting compound (II) from diisopropyl ether before it undergoes oxidation.

Therefore, it is a first object of the present invention to provide the progesterone of formula (I) 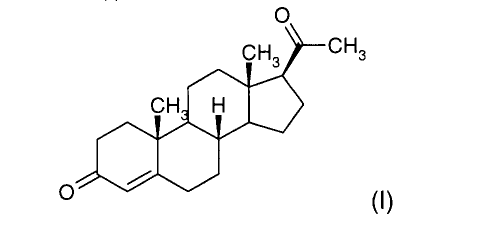 which is substantially free of Δ¹⁴-pregnene-3,20-dione, of formula (III)

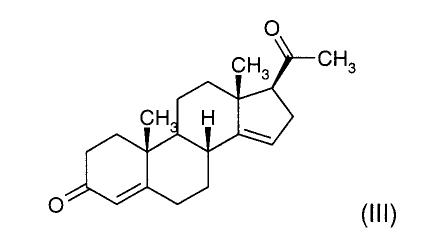

Further, it is a second object of the present invention to provide a method for obtaining the progesterone of formula (I) 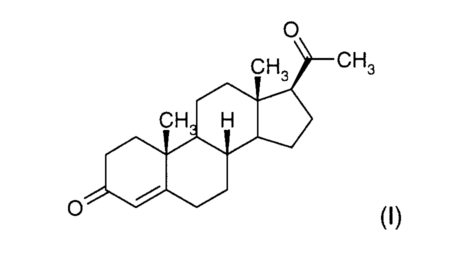 which is substantially free of Δ¹⁴-pregnene-3,20-dione of formula (III), by Oppenhauer oxidation of Δ⁵-pregnen-3β-ol-20-one of formula (II) 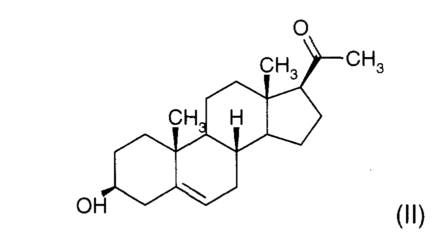 characterized in that the said compound of formula (II) is recrystallized from diisopropyl ether before it undergoes said oxidation.

## Claims

1. Progesterone of formula (I) 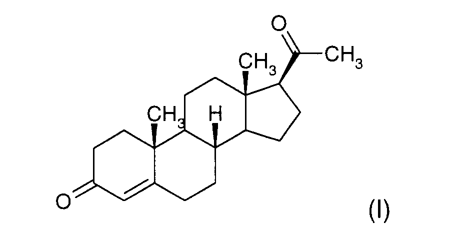 which is substantially free of Δ¹⁴-pregnene-3,20-dione of formula (III)

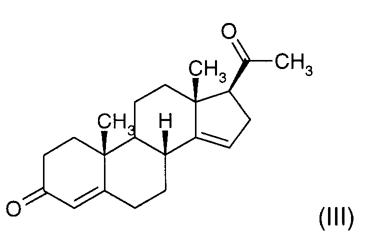

2. Method for obtaining the progesterone of formula (I) 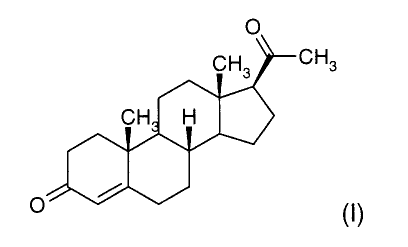 which is substantially free of Δ¹⁴-pregnene-3,20-dione of formula (III), by Oppenhauer oxidation of Δ⁵-pregnen-3β-ol-20-one of formula (II) 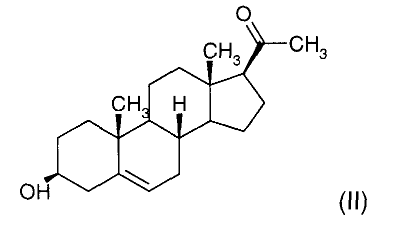 **characterized in that** the said compound of formula (II) is recrystallized from diisopropyl ether before it undergoes said oxidation.
